# EUROPÄISCHE PATENTANMELDUNG

(11) **EP 0 732 103 A2**
(43) Veröffentlichungstag der Anmeldung: **18.09.1996**
(21) Anmeldenummer: 96810096.6
(22) Anmeldetag: 19.02.1996
(51) Int. Cl.: A61K 35/78

(54) **Verfahren zur Isolierung von Wirkstoffen aus frischen Heilpflanzen bei tiefen Temperaturen durch ein Mizellen/Liposomengemisch**

(30) Priorität: 14.03.1995 CH 718/95
(71) Anmelder: Weis, Ingrid, 4148 Pfeffingen (CH)
(72) Erfinder: Weis, Ingrid, 4148 Pfeffingen (CH)

(57) **Zusammenfassung**

Beschrieben wird ein neues Verfahren zur Isolierung von Wirkstoffen aus frischen Heilpflanzen in flüssigem Stickstoff mit einem in situ gebildeten Mizellen/Liposomengemisch, welches an ein hydrophiles/hydrophobes Trägermaterial adsorbiert wird, um ein rieselfähiges Pulver zu erhalten, welches als Arzneimittel verarbeitet werden kann. Dieses Verfahren dient zur Herstellung und Intensivierung von Heilmitteln auf pflanzlicher und rein biologischer Basis. Bekannte Heilpflanzen, deren therapeutische Wirkungen belegt sind, können mit diesem Verfahren mit ihrer gesamten Wirkstoffbreite angewendet werden, ohne dass ihre Wirkstoffkomponenten durch Erhitzen, alkoholische Extraktionen, Trocknen oder Vergären verändert oder zerstört werden.

## Beschreibung

Beschrieben wird ein neues Verfahren zur Isolierung von Wirkstoffen aus frischen Heilpflanzenpflanzen in flüssigem Stickstoff mit einem in situ gebildeten Mizellen/Liposomengemisch, welches an ein hydrophiles/hydrophobes Trägermaterial absorbiert wird, um dann in Kapsel abgefüllt zu werden. Dieses Vefahren dient zur Herstellung und Intensivierung von Heilmitteln auf pflanzlicher und rein biologischer Basis. Bekannte Heilpflanzen, deren therapeutische Wirkungen belegt sind können mit diesem Verfahren mit ihrer gesamten Wirkstoffbreite angewendet werden, ohne dass ihre Wirkstoffkomponenten durch Erhitzen, alkoholische Extraktionen, Trocknen oder Vergären verändert oder zerstört werden.

Frisch geerntete grüne Blätter oder Blüten von einer Heilpflanze werden je nach Bedarf zuerst apparativ klein geschnitten oder die ganzen Blätter in flüssigem Stickstoff getaucht, dabei werden sie auf ungfähr -190°C abgekühlt. Durch das schockartige Abkühlen platzen die Pflanzenzellen auf. Der Inhalt der Pflanzenzellen wird dann durch in situ gebildeten Liposomen/Mizellen eingefangen. Die Mizellen/Liposomen werden durch Zugabe eines Mizellenbildners geformt, wie z.B. ein Speiseöl, Lezithine oder Polyäthylenglykolle. Diese Zugabe erfolgt zu den tiefgekühlten zu Pulver zerschnittenen Blättern bei gleichzeitigem intensiven Rühren. Die frei gewordenen Inhaltsstoffe der Pflanzenzellen werden so von den Mizellen/Liposomen eingeschlossen. Um eine möglichst quantitative Aufnahme der Wirkstoffe der Pflanzenzellen in die Mizellen/Liposomen zu erreichen wird dieser Vorgang 3-4 mal wiederholt.

Das Mizellen/Liposomengemisch wird dann an einem hydrophoben/hydrophilen Trägergemisch absorbiert, wie z.B. hochdisperses,amorphes Siliziumdioxid,, wobei ein rieselfähiges Pulver entsteht, welches in Kapseln abgefüllt werden kann. Diese Verfahren stellt sicher, dass alle Wirkstoffe der Pflanze in ihrer natürlichen biologischen Aktivität und Konformation erhalten bleiben. Dies gilt für die dabei freigesetzten Proteine, welche als Enzyme wirken können, ebenso wie für die freigesetzten Vitamine und die relevanten medizinischen Wirkstoffe, die die Pflanze synthetisiert hat. Da keine Auftrennung in einzelne Wirkkomponenten erfolgt, kann somit die Heilpflanze als Gesamtheit medizinisch-therapeutisch eingesetzt werden, ohne Verlust an Wirkstoffen. Eine neue Art der Verabreichung von pflanzlichen Wirkstoffen stellt das Mizellen/Liposomen Gemisch mit den natürlichen, biologisch hochwirksamen Wirkstoffen dar. Kommen die Mizellen/Liposomen in den Magen oder Darm, binden sie sich sofort an dessen Zellen, fusionieren mit ihnen, um so ihren Inhalt direkt in diese Zielzellen weiter zu geben.

### Beispiel

Petroselinum crispum (Petersilie) wird nach der Ernte gewaschen und überschüssiges Wasser abgetropft. Von dieser Petersilie wird 1 kg in ein 5 l Dewargefäss gefüllt und mit 2 l flüssigen Stickstoff beschichtet. Das Dewargefäss wird während 2 Stunden geschlossen gehalten. Während dieser Zeit kühlt die Petersilie bis auf ungefähr -180°C ab. Bei diesen schockartigen Abkühlungsprozess platzen die pflanzlichen Zellen, die grünen Blätter werden glasig brüchig. Diese tief gefrorene Petersilie kann nun mit einem elektrischen Zerschneider zu feinem grünen Pulver zerkleinert werden. Nicht zerkleinerte Teile wie z.B. lange Stengel werden durch ein Sieb von dem feinem Pulver bei tiefer Temperatur getrennt. Dann werden 0.8 kg Rapsöl zu dem Petersilien Pulver dazu gegeben, und mit einem Emulgationsrühmesser solange gerührt, bis ungefähr 4°C und ein hellgrünes Emulgat erreicht sind. Diese Emulgat wird anschliessend mit 1L flüssigen Stickstoff versetzt um es wieder schockartig abkühlen zu lassen. Das gefrorene Emulgat lässt man langsam auftauen, während des Auftauens wird mit dem Emulgationsrühmesser gerührt, bis wieder ungefähr 4°C erreicht sind. Dieser Vorgang wird noch 2 mal wiederholt. Danach wird das Petersilien Emulgat mit dem gleichen Gewicht an Aerosil R972 als Absorbtionsmaterial (Degussa AG, GB AC, Postfach 1105 33, D-6000 Frankfurt 11 ) unter langsamen Rühren gemischt, dabei entsteht eine feste grüne Paste. Diese grüne Paste wird mit dem 4-5fachen Gewicht an Arosil R974 zu einem feinem streufähigen Pulver verrührt. Dieses Pulver wird in Kapseln zu ungefähr 25 mg abgefüllt.

Die Wirkstoffe wie Vitamin A, Vitamin C, Apiol, Myristicin, Limonen, Eugenol, Pinen, Cumarine, Bergapten, Flavonoide, Apigenin sind aus den Zellen frei gesetzt, durch das Oel/Wassergemisch gelöst und an Aerosil absorbiert. Dies wird durch ein Dünnschicht Diagramm (DC) nachgewiesen. Für das DC werden 20 mg des Emulgats auf eine UV-beschichtete Silicagel Platte aufgetragen und in Toluol entwickelt. Die aufgetrennten Wirkstoffe werden im UV-Licht identifiziert. Ein photometrisches Spektrum einer alkoholischen Lösung des Emulgates im sichtbaren- und im UV-Bereich dient als Identifikation.

Die Wirkstoffe von Petroselinum crispum besitzen ein bekanntes breites therapeutisches Spektrum, wie harntreibend, menstruationsfördernd, rheumalindernd, antimikrobiell, Muskeltonus und Krämpfe lindernd, Darmtätigkeit steigernd, beseitigt Blähungen und Koliken. Dieses Präparat darf nicht von Scwangeren eingenommen werden, aber bei stillenden Müttern wird der Milchfluss gesteigert. Da viel Chlorophyll in diesem Präparat vorhanden ist, dient es ebenso zum Reinhalten des Atems.

## Patentansprüche

1. Verfahren zur Isolierung von Wirkstoffen aus frischen Heilpflanzen bei tiefen Temperaturen durch ein Mizellen/Liposomengemisch, dadurch gekennzeichnet, dass frische Heilpflanzen in flüssigem Stickstoff abgekühlt und zerhackt, und mit 80% des Gewichtes der Pflanzen mit einem Mizellenbildner versetzt und unter langsamem Auftauen so intensiv gerührt werden, dass eine Emulsion entsteht, diese Operation wird mehrmals wiederholt, so, dass die pflanzlichen Zellen platzen und die frei werdenden Wirkstoffe durch die gebildeten Mizellen/Lposomen gelöst werden, und das entstandene Gemisch wird an ein Trägermaterial absorbiert, so, dass ein trockenes rieselfähiges Pulver entsteht, welches in Kapseln abgefüllt werden kann.
